# EUROPEAN PATENT SPECIFICATION

(11) **EP 1 616 962 B1**
(45) Date of publication and mention of the grant of the patent: **06.02.2019**
(21) Application number: 04725536.9
(22) Date of filing: 02.04.2004
(51) Int. Cl.: C12P 13/02, C08F 20/56

(54) **ENZYMATIC PROCESS FOR PRODUCING HIGH-QUALITY ACRYLAMIDE POLYMER**
ENZYMATISCHES VERFAHREN ZUR HERSTELLUNG EINES QUALITATIV HOCHWERTIGEN ACRYLAMIDPOLYMERS
PROCEDE ENZYMATIQUE DE FABRICATION D'UN POLYMERE ACRYLAMIDE DE HAUTE QUALITE

(30) Priority: 10.04.2003 JP 2003106894
(43) Date of publication of application: 18.01.2006
(73) Proprietor: Mitsubishi Chemical Corporation, Tokyo 100-8251 (JP)
(72) Inventor: MURAO, Kozo, Chiyoda-ku, Tokio 100-8251 (JP); ISHII, Katsuo, Chiyoda-ku, Tokio 100-8251 (JP); KANO, Makoto, Chiyoda-ku, Tokio 100-8251 (JP); BANBA, Hiroyasu, Chiyoda-ku, Tokio 100-8251 (JP)
(74) Representative: Hoffmann Eitle
(86) International application number: PCT/JP2004/004847
(87) International publication number: WO 2004/090148

(56) References cited:
- EP-A1- 0 635 484
- JP-A- 9 227 478
- JP-A- 10 316 714
- JP-A- 55 020 791
- JP-A- 2002 080 443
- US-A- 6 043 061
- HWANG J S ET AL: "BIOTRANSFORMATION OF ACRYLONITRILE TO ACRYLAMIDE USING IMMOBILIZED WHOLE CELLS OF BREVIBACTERIUM CH1 IN A RECYCLE FED-BATCH REACTOR", BIOTECHNOLOGY AND BIOENGINEERING, WILEY & SONS, HOBOKEN, NJ, US, vol. 34, 1 January 1989 (1989-01-01), pages 380-386, XP001040408, ISSN: 0006-3592, DOI: DOI:10.1002/BIT.260340311

## Description

### Technical Field

The present invention relates to a method for producing a high-quality acrylamide polymer preferable for flocculants, thickeners for papermaking, using an enzymatic method.

### Background Art

Acrylamide polymers are utilized for flocculants, thickeners for papermaking, and the like. For any use, an acrylamide polymer having a high molecular weight, which are highly soluble and almost colorless, is desired.

When using acrylamide polymers for flocculants, for example, an acrylamide polymer having a low molecular weight causes problems in terms of flocculation ability. Also, when using an acrylamide polymer having poor solubility, prolonged processing time becomes a problem. Further, when using acrylamide polymers for thickeners for papermaking, an acrylamide polymer having poor solubility causes defects such as fisheyes on paper. In addition, regardless of the use thereof, an almost colorless acrylamide polymer is required.

Under such circumstances, to improve the solubility of an acrylamide polymer, the removal of impurities in acrylonitrile to produce acrylamide followed by production of an acrylamide polymer using the obtained acrylamide as a starting material has been discussed. Also, it has been known that an enzymatic method is preferably adopted for a method for producing acrylamide.

J. S. Hwang et al., Biotechnology and Bioengineering, vol. 34, 1989, pages 380-386, discloses a process for producing an acrylamide polymer wherein acrylonitrile is enzymatically hydrated using immobilized cells of Brevibacterium CH1 to produce acrylamide. Subsequently the acrylamide is polymerized.

JP Patent Publication (Kokai) No. 10-316714 A (1998) (Patent document 1) discloses that a high-quality acrylamide polymer can be produced using acrylamide as a starting material obtained by utilizing an enzymatic method. In addition, JP Patent Publication (Kokai) No. 11-123098 A (1999) (Patent document 2) discloses that the use of acrylonitrile as a starting material, which contains hydrogen cyanide at a low concentration, can suppress decreases in enzyme activity upon the production of acrylamide by an enzymatic method.

Further, JP Patent Publication (Kokai) No. 9-227478 A (1997) (Patent document 3) discloses that acrylamide is produced from acrylonitrile containing oxazole at a concentration of 1 ppm or less using a copper catalyst, and that the produced acrylamide is polymerized to yield an acrylamide polymer. In such document (paragraph nos. 0009 to 0011 in the Japanese text), it is described that acrylonitrile containing few impurities such as hydrogen cyanide is preferably used.

In the above references, conditions of processes for producing acrylamide have been discussed; however, no discussion has been made in view of the color of an acrylamide polymer. There has been no elucidation of how various types of impurities (such as acetone, methacrylonitrile, acetaldehyde, acetonitrile, benzene, propionitrile, and acrolein) contained in acrylonitrile affect the color of an acrylamide polymer.

The object of the present invention is to provide a method for producing an acrylamide polymer, that is almost colorless when in the form of an aqueous solution and that is white when in powdered form.
Patent document 1
   JP Patent Publication (Kokai) No. 10-316714 A (1998)
Patent document 2
   JP Patent Publication (Kokai) No. 11-123098 A (1999)
Patent document 3
   JP Patent Publication (Kokai) No. 9-227478 A (1997)

### Disclosure of the Invention

As a result of intensive studies to address the above problems, the inventors have completed the present invention by finding that, when converting acrylonitrile to acrylamide to produce an acrylamide polymer from the acrylamide, hydrogen cyanide, which has not been recognized as an impurity in acrylonitrile, significantly affects physical properties of an acrylamide polymer, and particularly the color thereof, in addition to oxazole contained as an impurity in acrylonitrile. In other words, the present invention includes the following:
(1) A method for producing an acrylamide polymer comprising steps of: hydrating acrylonitrile containing oxazole at a concentration of 5 mg/kg or less and hydrogen cyanide at a concentration of 1 mg/kg or less by an enzymatic method to yield acrylamide; and polymerizing monomers containing the acrylamide.
(2) The method for producing an acrylamide polymer according to (1) above, wherein, in the reaction step of hydrating acrylonitrile using an enzymatic method, the reaction is carried out until the concentration of acrylamide generated in the reaction solution becomes 30% by mass or more.
(3) The method for producing an acrylamide polymer according to (1) or (2) above, wherein the enzymatic method is carried out using microbial cells as catalysts.

### Best Mode for Carrying Out the Invention

Acrylonitrile that can be used in the method of the present invention is acrylonitrile containing oxazole at a concentration of 5 mg/kg or less and hydrogen cyanide at a concentration of 1 mg/kg or less.

The expression "acrylonitrile containing oxazole at a concentration of 5 mg/kg or less" indicates acrylonitrile that contains oxazole at a concentration of 5 mg or less per 1 kg of acrylonitrile.

Generally commercially available acrylonitrile contains 1 mg/kg to 100 mg/kg oxazole. Thus, when the concentration of oxazole in acrylonitrile is 5 mg/kg or more, oxazole is removed or the oxazole content is reduced. Removal of oxazole or reduction of the oxazole content in acrylonitrile can be performed by ion exchange resin treatment, purifying distillation, and the like. Particularly, a method where a strong acid ion exchange resin is made to come into contact with acrylonitrile is convenient and economically favorable, see EP-A-635484.

The concentration of oxazole in acrylonitrile can be determined by, for example, a capillary gas chromatograph equipped with a DB 225 column (Agilent Technologies) and the like.

The expression "acrylonitrile containing hydrogen cyanide at a concentration of 1 mg/kg or less" indicates acrylonitrile that contains hydrogen cyanide at a concentration of 1 mg or less per 1 kg of acrylonitrile. Generally commercially available acrylonitrile contains 0.1 mg/kg to 5 mg/kg hydrogen cyanide. Thus, when the concentration of hydrogen cyanide in acrylonitrile is 1 mg/kg or more, hydrogen cyanide is removed or the hydrogen cyanide content is reduced.

Examples of a method for removing hydrogen cyanide or reducing the hydrogen cyanide content in acrylonitrile include a method using anion exchange resin, a method for extracting hydrogen cyanide using an alkaline solution (JP Patent Publication (Kokai) No. 2001-288256 A), and a method for adding hydrogen cyanide to acrylonitrile with addition of alkalis (JP Patent Publication (Kokai) No. 11-123098 A (1999)).

The concentration of hydrogen cyanide in acrylonitrile can be determined by methods such as a method using a capillary gas chromatograph equipped with, for example, an NPD detector and a DB 225 column (Agilent Technologies), and a method of titration with a silver nitrate aqueous solution following extraction in an alkaline solution (ASTM E1178-87). In the present invention, "the concentration of hydrogen cyanide in acrylonitrile" indicates values determined by ASTM (E1178-87).

Subsequently, the thus prepared acrylonitrile containing oxazole at a concentration of 5 mg/kg or less and hydrogen cyanide at a concentration of 1 mg/kg or less is hydrated (hydrolyzed) by an enzymatic method so as to yield acrylamide. Herein, the expression "acrylonitrile is hydrated (hydrolyzed) by an enzymatic method so as to yield acrylamide" indicates that acrylamide is produced from acrylonitrile by catalysis of an enzyme capable of hydrating (hydrolyzing) acrylonitrile, thereby converting the acrylonitrile to acrylamide.

An enzyme that is used in the above hydration reaction may be any enzyme that has the conversion ability described above. A preferred example thereof is nitrile hydratase. Nitrile hydratase is an enzyme that converts a nitrile compound to the corresponding amido compound. Known examples thereof are derived from microorganisms belonging to the genera *Bacillus, Bacteridium, Micrococcus, Brevibacterium, Corynebacterium, Nocardia, Pseudomonas, Rhodococcus, Microbacterium, Rhodococcus* (the species *Rhodococcus rhodochrous*), *Fusarium,* and *Agrobacterium.*

Further, a transformant that may be used herein is prepared by obtaining a nitrile hydratase gene derived from the above microorganisms and then introducing the gene directly, or an artificially improved gene, into any host using a conventional technique (Molecular Cloning 2nd Edition, Cold Spring Habor Laboratory Press, 1989).

Examples of the above transformant may include *Escherichia coli* MT10770 (FERM P-14756) (JP Patent Publication (Kokai) No. 8-266277 A (1996)) that has been transformed with nitrile hydratase of microorganisms of the genus *Achromobacter, Escherichia coli* MT10822 (FERM BP-5785) (JP Patent Publication (Kokai) No. 9-275978 A (1997)) that has been transformed with nitrile hydratase of microorganisms of the genus *Pseudonocardia,* or microorganisms transformed with nitrile hydratase (JP Patent Publication (Kokai) No. 4-211379 A (1992)) of the species *Rhodococcus rhodochrous.*

Examples of the form in use of nitrile hydratase that may be used herein include a culture solution obtained by culturing the above nitrile hydratase-producing organisms and the like according to a conventional method, resting cells or immobilized cells isolated from the culture solution, crude or purified enzymes of nitrile hydratase extracted from the resting cells or the like, and crude or purified enzymes immobilized on carriers (such as polyacrylamide gel, alginate, and carrageenan).

Hydration reaction of acrylonitrile to form acrylamide by an enzymatic method can be carried out under conditions of a conventional technique at ordinary temperature. An example of an enzymatic method that can be carried out will be described below.

Nitrile hydratase-producing organisms are cultured in a medium with addition of carbon sources (saccharides such as glucose), nitrogen sources (inorganic nitrogen sources such as ammonium sulfate, ammonium chloride, and ammonium nitrate, and organic nitrogen such as yeast extract, peptone, and meat extract), and optionally, inorganic salts, metal salts, vitamins, or the like, at 20°C to 40°C (pH 5 to 9). The culture may be carried out by shake culture or rotation culture in an adequate manner.

After the termination of the culture, microbial cells are washed with a phosphate buffer or the like so as to prepare a cell suspension thereof. When preparing immobilized cells, monomers such as acrylamide are added to the cell suspension for polymerization, thereby allowing the immobilized cells to be obtained therefrom.

Then, water and immobilized cells such as those obtained above are put in a reaction vessel, the solution thereof is adjusted to have a pH level of 5 to 9.5 and a temperature of 5°C to 50°C, and then acrylonitrile is added thereto to serve as a substrate. It is preferable for acrylonitrile to be added sequentially to the reaction solution so that the concentration of acrylonitrile in the reaction solution falls within the range of 0.1% by mass to 10% by mass. Depending on the progress of an enzymatic reaction, enzymes may be added to the reaction solution in an adequate manner. This enzymatic reaction is allowed to continue until acrylonitrile becomes undetectable in the reaction solution. Preferably, the reaction is carried out until the concentration of acrylamide accumulated in the reaction system becomes 30% by mass or more, and particularly preferably, 40% by mass to 60% by mass. When the concentration of acrylamide arrives at the desired level, addition of acrylonitrile is stopped, and then the reaction is allowed to continue until acrylonitrile in the reaction solution becomes undetectable.

As described above, when producing acrylamide by an enzymatic method, it is more economical to allow acrylamide to be accumulated in a reaction solution at a higher concentration so that a high-quality acrylamide polymer can be produced.

Next, acrylamide that has been produced according to the above method is subjected to a polymerization reaction. Acrylamide may be used in the form of the acrylamide aqueous solution directly after the hydration reaction. If necessary, acrylamide may be used after being subjected to concentration operations such as an evaporative concentration operation and purification operations such as an activated carbon treatment, an ion exchange treatment, and a filtration treatment.

Examples of production of an acrylamide polymer will be described below.

In the present invention, the term "acrylamide polymer" indicates an acrylamide homopolymer or a copolymer made up of acrylamide and at least one unsaturated monomers copolymerizable therewith. Examples of such unsaturated copolymerizable monomers that may be used herein include water-insoluble and hydrophobic monomers such as acrylonitrile and styrene, as long as solubility of the obtained polymers is maintained, in addition to the following examples: (meth)acrylamide derivatives such as methacrylamide, 2-acrylamide-2-methylpropane sulfonic acid (sulfonate), N-methylolacrylamide, dimethylaminopropyl acrylamide and quaternary ammonium salts thereof, and N,N-dimethyl acrylamide; acids such as (meth)acrylate, vinylsulfonic acid, allylsulfonic acid, and styrenesulfonic acid, and water-soluble salts thereof; lower acrylic ester derivatives of (meth)acrylic acids such as ethyl acrylate, methyl acrylate, and hydroxypropyl methacrylate; alkyl(methyl or ethyl)aminoalkyl(ethyl or propyl)esters of (meth)acrylic acids such as N,N-dimethylaminoethyl methacrylate, N,N-diethylaminoethyl methacrylate, N,N-dimethylaminoethyl acrylate, and quaternary ammonium salts thereof, and quaternary ammonium derivatives thereof; 2-vinylimidazoline and 2- vinylpyrimidine, and quaternary ammonium derivatives thereof; and N-vinylacetamide, vinyl acetate, and vinyl pyrrolidone.

In the method of the present invention, the concentration of acrylamide upon polymerization in an aqueous solvent or the total concentration of acrylamide and monomers copolymerizable therewith is generally within the range of 10% by mass to 90% by mass, and preferably of 20% by mass to 80% by mass. When the concentration is 10% by mass or more, an acrylamide polymer having a high molecular weight can be obtained. When the concentration is 90% by mass or less, crosslinking reactions during polymerization can be prevented so that reduction in solubility or insolubilization of the polymer can be suppressed.

A method of polymerization that may optionally be adopted in the present invention is any method where polymerization is carried out in an aqueous solvent. Examples thereof include, but are not limited to, methods of adiabatic polymerization and sheet polymerization while removing generated heat via a belt. The temperature for polymerization is within the range of 0°C to 120°C, and preferably, of 10°C to 90°C.

A polymerization initiator that may be used is a polymerization initiator that is conventionally known in general. Examples thereof include: peroxides such as potassium persulfate, ammonium persulfate, benzoyl peroxide, hydrogen peroxide, and t-butylhydroperoxide; azo compounds such as azobisisobutyronitrile; photodegradation-type polymerization initiators such as benzoin ethyl ether; and reducers such as sodium hydrogen sulfite, sodium sulfite, sodium hydrosulfite, triethanolamine, and ferrous sulfate, which form polymerization initiators with the above peroxides by redox reaction. At least one of these polymerization initiators may be used according to a conventional technique.

The obtained polymer may be shredded using a mincer such as a meat chopper, dehydrated, and further disrupted using a grinder according to a conventional technique so as to yield a polyacrylamide dry product in powdered form. Examples of drying equipment that may be used in an adequate manner include, but are not particularly limited to, a tray-type dryer, a belt dryer, a rotary dryer, a fluid dryer, a infrared dryer, and a high-frequency dryer.

When the acrylamide polymer obtained by the above method is dissolved at a concentration of 1% by mass in a 4% by mass saline solution for viscosity measurement at 25°C using a type B viscometer and a Rotor No. 3 (rotation speed of rotor: 6 rpm), the viscosity thereof is 2,000 mPa·s or more, and preferably, 3,000 mPa·s or more (where the viscosity of 2,000 mPa·s corresponds to an acrylamide polymer having a molecular weight of approximately 10,000,000). The color of acrylamide polymer powder obtained by the method of the present invention is white. When the polymer powder is dissolved in ion exchange water at a concentration of 0.1% by mass and the aqueous solution thereof is filtered with an 80-mesh woven metal filter for visual observation of the amount of gelatinous insoluble matters that have remained on the filter, insoluble matter can scarcely be observed. Therefore, the acrylamide polymer obtained by the method of the present invention can be used preferably for flocculants, thickeners for papermaking, and the like.

The acrylamide polymer obtained above according to the method of the present invention is almost colorless when in the form of an aqueous solution and white in powdered form. The colors of acrylamide polymer powders can be evaluated by placing approximately 1 g of the powders separately on white paper for color comparison.

The present invention will be described more specifically by the following examples. In addition, the term "%" denotes "% by mass" in Examples and Comparative examples below.

### <Example 1>

### (1) Preparation of immobilized cells

A *Rhodococcus rhodochrous* J-1 strain (FERM BP-1478) having nitrile hydratase activity was aerobically cultured in a medium (pH 7.0) containing 2% glucose, 1% urea, 0.5% peptone, 0.3% yeast extract, and 0.05% cobalt chloride. After the termination of the culture, the cultured microbial cells were recovered by centrifugation and washed with a 50 mM phosphate buffer (pH 7.0). Then, the buffer was added to the washed cells, thereby obtaining a cell suspension thereof (20% when converted into dry cell weight). To 500 g of the cell suspension, 500 g of a monomer mixture aqueous solution containing acrylamide, methylene bisacrylamide, and 2-dimethylaminopropyl methacrylamide at a concentration of 20%, 2% and 2%, respectively, was added so as to allow suspension to be performed well. Then, 2 g of 5% ammonium persulfate and 2 g of 50% N,N,N,N-tetramethylethylenediamine were added to the suspension for polymerization and gelatinization. The resultant was cut into an approximately 1-mm cube, and then washed 5 times with 1 l of 0.5% sodium sulfate, thereby obtaining immobilized cell particles that could serve as a catalyst for the production of acrylamide.

### (2) Preparation of acrylonitrile

Acrylonitrile (Dia-Nitrix Co., Ltd.) containing oxazole at a concentration of 10 mg/kg and hydrogen cyanide at a concentration of 0.7 mg/kg (measured as defined by ASTM E1178-87) was treated with a strong acid ion exchange resin, Amberlyst 15 Wet (Organo Corporation), so as to prepare acrylonitrile containing oxazole at a concentration of 5 mg/kg or less (undetected by a gas chromatograph equipped with a FID detector and a column DB 225, Agilent Technologies) and hydrogen cyanide at a concentration of 0.7 mg/kg. Then the thus obtained acrylonitrile was used.

### (3) Production of acrylamide by an enzymatic method

Three thousand and two hundred grams of a 0.2 g/l sodium acrylate aqueous solution was put in a separable flask with an internal volume of 5 liters. Five grams of the immobilized cell particles (5 g) prepared in (1) above were added thereto. The solution was agitated while maintaining pH level of 7.0 and a temperature of 15°C. To this solution, acrylonitrile was sequentially fed so as to keep the concentration of acrylonitrile at 2%, and then an enzymatic reaction was performed until the acrylamide concentration became 47.3%. During the reaction, at each time when progress of the reaction become unobservable, 1 g of immobilized cells were added. Thereafter, feeding of acrylonitrile was stopped, and then the reaction was allowed to continue at a temperature of 20°C until acrylonitrile in the reaction solution became undetectable.

After the termination of the reaction, a 50% acrylamide aqueous solution was obtained by removing the immobilized cells using a 180-mesh woven metal filter.

### (4) Production of acrylamide polymer

The 50% acrylamide aqueous solution (348 parts) and the 98% by mass acrylic acid (2 parts) obtained in (3) above were weighed and put in a beaker (1 liter), and then ion exchange water (400 parts) was added thereto. The solution was neutralized with the addition of sodium hydroxide. Further, ion exchange water was added thereto to prepare 797 parts thereof as a total. The obtained solution was adjusted to have a temperature of 10°C and transferred to a Dewar flask (1 liter). The solution was purged with nitrogen gas for 30 minutes, and then, as polymerization initiators, a 10% 2,2'-azobis(2-amidinopropane)dihydrochloride aqueous solution (1.5 parts), a 0.2% sodium hydrosulfite aqueous solution (1 part), and a 0.2% t-butylhydroperoxide aqueous solution (0.5 parts) were added thereto so as to initiate polymerization. Polymerization intermittently proceeded and the peak temperature reached approximately 74°C. 30 minutes after reaching the peak temperature, the resulting polymer was collected, cut into a 5-cm cube using scissors, and shredded using a mincer (meat chopper) with a 5-mm diameter mesh plate. The shredded gelatinous polymers were dehydrated at 60°C for 16 hours using a hot air dryer and disrupted using a Wiley grinder with a 2-mm diameter mesh plate. Subsequently, the disrupted particles thereof were sieved at a particle diameter of 0.15 to 1.0 mm, thereby obtaining copolymer powder containing acrylamide and acrylic acid.

### (5) Evaluation of polymer

The polymer powder obtained in (4) above was dissolved in a 4% by mass saline solution at a concentration of 1% by mass, and 1% salt viscosity thereof was determined at 25°C using a type B viscometer.

Further, to examine the water solubility thereof, the polymer powder was dissolved in 5 kg of ion exchange water at a concentration of 0.1% by mass, and then filtered with an 80-mesh woven metal filter. The amount of insoluble gelatinous matter that had remained on the filter was visually evaluated. Also, regarding the polymer color, the polymer powder was visually observed.

### <Comparative example 1>

Copolymer powder containing acrylamide and acrylic acid was prepared in the same manner as in the case of Example 1 except for using, as a starting material, acrylonitrile containing oxazole at a concentration of 10 mg/kg and hydrogen cyanide at a concentration of 0.7 mg/kg, instead of acrylonitrile containing oxazole at a concentration of 5 mg/kg or less and hydrogen cyanide at a concentration of 0.7 mg/kg. Then, the obtained polymer was evaluated.

### <Comparative example 2>

Copolymer powder containing acrylamide and acrylic acid was prepared in the same manner as in the case of Example 1 except for using, as a starting material, acrylonitrile containing oxazole at a concentration of 10 mg/kg and hydrogen cyanide at a concentration of 5 mg/kg, instead of acrylonitrile containing oxazole at a concentration of 5 mg/kg or less and hydrogen cyanide at a concentration of 0.7 mg/kg. Then, the obtained polymer was evaluated.

### <Comparative example 3>

Copolymer powder containing acrylamide and acrylic acid was prepared in the same manner as in the case of Example 1 except for using, as a starting material, acrylonitrile containing oxazole at a concentration of 5 mg/kg or less and hydrogen cyanide at a concentration of 5 mg/kg, instead of acrylonitrile containing oxazole at a concentration of 5 mg/kg or less and hydrogen cyanide at a concentration of 0.7 mg/kg. Then, the obtained polymer was evaluated.

Table 1 shows physical properties of the copolymers containing acrylamide and acrylic acid obtained in Example 1 and Comparative examples 1-3.

**Table 1**

| | Acrylonitrile Used | | Polymer Aqueous Solution | | Color Polymer Powder |
|---|---|---|---|---|---|
| | Oxazole Concentration [mg/kg] | Hydrogen Cyanide Concentration [mg/kg] | 1% Salt Viscosity [mPa·s] | Solubility | |
| Example 1 | ≤5 | 0.7 | 3600 | + | + |
| Comparative Example 1 | 10 | 0.7 | 3620 | - | ± |
| Comparative Example 2 | 10 | 5 | 3550 | - | - |
| Comparative Example 3 | ≤5 | 5 | 3580 | - | ± |

| | | | | | |
|---|---|---|---|---|---|
| Solubility: +: Almost no gelatinous substance; -: Gelatinous substance observed Color: +: White; ±: Slightly yellow; -: Yellow | | | | | |

According to the above results, the acrylamide polymer produced by the method of the present invention has good solubility and excellent color.

### <Example 2>

### (1) Production and evaluation of acrylamide polymer

The 50% acrylamide aqueous solution (192 parts) prepared according to Example 1, ion exchange water (8 parts) and nitrilotrispropionic acid amide (0.18 parts), which is a chain transfer agent, were mixed together and adjusted to have a pH level of 10 with addition of a 0.1 N NaOH aqueous solution. Then, a methanol solution (0.5 parts) containing benzoin ethyl ether as a photoinitiator dissolved therein at a concentration of 1% was added to the above solution. The obtained polymerization solution was subjected to nitrogen exchange while shielding light. An SUS container was used for polymerization and a glass plate was installed on top thereof. The container was placed in the bath at a temperature of 20°C under a nitrogen atmosphere, and then a polymerization solution that had been subjected to nitrogen exchange was fed thereto so as to obtain a 5-mm thick sheet. The container was irradiated from the top using a chemical lamp (FL-20S-BL, TOSHIBA) to perform photo-initiated sheet polymerization. The photo polymerization was performed under the conditions of light irradiation at a light intensity of 1.0 W/m² for 40 minutes, and further, of 40 W/m² for 30 minutes following the polymerization by the prior light irradiation.

The gelatinous polymer after polymerization was cut into a 2- to 3-mm cube using scissors and dehydrated at 60°C for 16 hours. The cut pieces thereof were disrupted using a Wiley grinder. The disrupted particles were sieved at a particle diameter of 0.15 to 1.0 mm, thereby obtaining acrylamide polymer powder.

The obtained acrylamide polymer was evaluated in the same manner as in the case of Example 1 (5).

### <Comparative example 4>

An acrylamide polymer was produced in the same manner as in the case of Example 2 except for using, as a starting material, acrylonitrile containing oxazole at a concentration of 10 mg/kg and hydrogen cyanide at a concentration of 0.7 mg/kg, instead of acrylonitrile containing oxazole at a concentration of 5 mg/kg or less and hydrogen cyanide at a concentration of 0.7 mg/kg.

### <Comparative example 5>

An acrylamide polymer was produced in the same manner as in the case of Example 2 except for using, as a starting material, acrylonitrile containing oxazole at a concentration of 10 mg/kg and hydrogen cyanide at a concentration of 5 mg/kg, instead of acrylonitrile containing oxazole at a concentration of 5 mg/kg or less and hydrogen cyanide at a concentration of 0.7 mg/kg.

### <Comparative example 6>

An acrylamide polymer was produced in the same manner as in the case of Example 2 except for using, as a starting material, acrylonitrile containing oxazole at a concentration of 5 mg/kg or less and hydrogen cyanide at a concentration of 5 mg/kg, instead of acrylonitrile containing oxazole at a concentration of 5 mg/kg or less and hydrogen cyanide at a concentration of 0.7 mg/kg.

Table 2 shows physical properties of the copolymers containing acrylamide and acrylic acid obtained in Example 2 and Comparative examples 4-6.

**Table 2**

| | Acrylonitrile Used | | Polymer Aqueous Solution | | Color of Polymer Powder |
|---|---|---|---|---|---|
| | Oxazole Concentration [mg/kg] | Hydrogen Cyanide Concentration [mg/kg] | 1% Salt Viscosity [mPa·s] | Solubility | |
| Example 2 | ≤5 | 0.7 | 4320 | + | + |
| Comparative Example 4 | 10 | 0.7 | 4280 | - | ± |
| Comparative Example 5 | 10 | 5 | 4350 | - | - |
| Comparative Example 6 | ≤5 | 5 | 4300 | - | - |

| | | | | | |
|---|---|---|---|---|---|
| Solubility: +: Almost no gelatinous substance; -: Gelatinous substance observed Color: +: White; ±: Slightly yellow; -: Yellow | | | | | |

According to the above results, it is understood that the acrylamide polymer produced by the method of the present invention has a high molecular weight, good solubility, and excellent color.

All publications, patents, and patent applications cited herein are incorporated herein by reference in their entirety.

### Industrial Applicability

According to the process of the present invention, a high-quality and very useful polyacrylamide polymer, which has a higher molecular weight, higher solubility, and more excellent color than those of conventional products, can be produced.

## Claims

1. A method for producing an acrylamide polymer comprising steps of: hydrating acrylonitrile containing oxazole at a concentration of 5 mg/kg or less and hydrogen cyanide at a concentration of 1 mg/kg or less by an enzymatic method to yield acrylamide; and polymerizing monomers containing the acrylamide.

2. The method for producing an acrylamide polymer according to claim 1, wherein, in the reaction step of hydrating acrylonitrile using an enzymatic method, the reaction is carried out until the concentration of acrylamide generated in the reaction solution becomes 30% by mass or more.

3. The method for producing an acrylamide polymer according to claim 1 or 2, wherein the enzymatic method is carried out using microbial cells as catalysts.

## Patentansprüche

1. Verfahren zur Herstellung eines Acrylamidpolymers, umfassend die Schritte: Hydratisieren von Acrylnitril, enthaltend Oxazol in einer Konzentration von 5 mg/kg oder weniger und Wasserstoffcyanid in einer Konzentration von 1 mg/kg oder weniger, durch ein enzymatisches Verfahren, um Acrylamid zu erhalten; und Polymerisieren von Monomeren, die das Acrylamid enthalten.

2. Verfahren zur Herstellung eines Acrylamidpolymers gemäss Anspruch 1, wobei in dem Reaktionsschritt, in dem Acrylnitril unter Verwendung eines enzymatischen Verfahrens hydratisiert wird, die Umsetzung solange durchgeführt wird, bis die Konzentration an in der Reaktionslösung erzeugtem Acrylamid 30 Masse-% oder mehr beträgt.

3. Verfahren zur Herstellung eines Acrylamidpolymers gemäss Anspruch 1 oder 2, wobei das enzymatische Verfahren unter Verwendung von mikrobiellen Zellen als Katalysator durchgeführt wird.

## Revendications

1. Procédé pour produire un polymère d'acrylamide comprenant les étapes suivantes : l'hydratation d'un acrylonitrile contenant un oxazole à une concentration de 5 mg/kg ou moins et du cyanure d'hydrogène à une concentration de 1 mg/kg ou moins par un procédé enzymatique pour produire un acrylamide ; et la polymérisation de monomères contenant l'acrylamide.

2. Procédé pour produire un polymère d'acrylamide selon la revendication 1, dans lequel, dans l'étape réactionnelle d'hydratation d'un acrylonitrile au moyen d'un procédé enzymatique, la réaction est effectuée jusqu'à ce que la concentration en acrylamide généré dans la solution réactionnelle soit de 30 % en masse ou plus.

3. Procédé pour produire un polymère d'acrylamide selon la revendication 1 ou 2, dans lequel le procédé enzymatique est effectué en utilisant des cellules microbiennes comme catalyseurs.
